# EUROPEAN PATENT APPLICATION

(11) **EP 4 548 763 A1**
(43) Date of publication of application: **07.05.2025**
(21) Application number: 23829369.0
(22) Date of filing: 19.01.2023
(51) Int. Cl.: A01N 1/02

(54) **HYPOTHERMIC LIVER PERFUSION PRESERVATION DEVICE AND METHOD**

(30) Priority: 29.06.2022 CN 202210758667
(71) Applicant: SHANGHAI GENEXT MEDICAL TECHNOLOGY CO., LTD, Shanghai 201114 (CN)
(72) Inventor: LUO, Ling, Shanghai 201114 (CN); WU, Yunlin, Shanghai 201114 (CN); YANG, Xiaolan, Shanghai 201114 (CN); MIAO, Fei, Shanghai 201114 (CN)
(74) Representative: WSL Patentanwälte Partnerschaft mbB
(86) International application number: PCT/CN2023/073031
(87) International publication number: WO 2024/001176

(57) **Abstract**

The present invention provides a liver hypothermic machine perfusion preservation device and a method. The device includes a perfusion loop and a controller, wherein the perfusion loop includes a main pump, a main reservoir, an auxiliary pump and an auxiliary reservoir. The main pump is connected to an inlet of the main reservoir and configured to pump a perfusate from an organ cassette into the main reservoir; an outlet of the main reservoir is connected to a portal vein and a hepatic artery of a liver, and the main reservoir is configured to supply the perfusate to the portal vein and the hepatic artery; and the auxiliary reservoir and the auxiliary pump provide an additional hepatic artery perfusion flow path. According to the present invention, for a poor internal environment of the hepatic artery, the main reservoir and the auxiliary reservoir cooperate with each other to carry out perfusion, so that the perfusion efficiency and effect of the hepatic artery can be improved. The device according to the present invention also has a molecular-sieve oxygen generation function, and a free adjustment range of oxygen concentration from 21% to 90% can be achieved by means of combined control of a molecular-sieve oxygen generation channel and an air channel, thus providing a more flexible solution for the perfusion.

## Description

### Technical Field

The present invention belongs to the field of biomedical instruments, and particularly relates to a hypothermic machine perfusion preservation device and a method for liver transplantation.

### Background Art

Organ transplantation refers to a surgical transfer of a viable isolated organ into a patient. Organ storage must be performed in a low-temperature environment with the temperature controlled at 2-8°C. A traditional organ preservation method is static cold storage. The longer the duration of static cold storage is, the higher the incidence of postoperative delayed graft function is. Moreover, the static cold storage makes it difficult to objectively evaluate an isolated organ. Compared with static cold storage, hypothermic machine perfusion of the isolated organ can significantly reduce the incidence of delayed graft function, which is beneficial to improve the long-term survival rate of the transplanted organ. In addition, machine perfusion parameters can assist in evaluating the quality of the transplanted organ, facilitating the full utilization of the isolated. An organ hypothermic perfusion preservation cassette is an apparatus for transfer, preservation and hypothermic machine perfusion of the isolated organ for transplantation. Organ hypothermic perfusion preservation can prolong the storage time of the isolated organ, evaluate the quality of the isolated organ and reduce the incidence of postoperative delayed graft function, and thus has now been widely used.

Taking liver transplantation as an example, in a living liver, blood flows in through a portal vein and a hepatic artery, mixes in sinus glands of the liver, and then flows out through hepatic veins. Portal venous blood accounts for 60% to 80%, with an average of 75% and a flow rate of about 1,100 ml per minute, and hepatic artery blood has a flow rate of about 350 ml per minute, accounting for 20% to 40% of the flow rate of the total liver blood, with an average of 25%. During perfusion preservation of an isolated liver, a perfusate flows in from the portal vein and the hepatic artery, and flows out through the hepatic veins to realise isolated perfusion of the liver. For example, a perfusion apparatus for perfusing an organ, disclosed in the prior art document CN 104619170 A, includes three different flow paths, namely a portal flow path connected to a portal vein of a liver, a hepatic flow path connected to a hepatic artery of the liver, and a bypass flow path which provides a return path to an organ basin. The portal flow path and the hepatic flow path are each provided with a pressure sensor, a flow sensor and a bubble sensor. In a perfusion mode, the perfusate enters the portal vein and the hepatic artery of the liver along the portal flow path and the hepatic flow path respectively. If a controller receives a signal from the pressure sensor that the pressure of a fluid is out of a predetermined range, the controller controls a pump to stop pumping the fluid. In addition, if the pressure in either or both of the portal flow path and the hepatic flow path exceeds a predetermined threshold, the apparatus may automatically stop and/or reduce the flow rate and/or pressure provided by the pump to prevent damage to the organ.

Typically, the flow into the portal vein and the hepatic artery is related to internal environments of vascular networks of the portal vein and the hepatic artery. Usually, the network of the portal vein is relatively unobstructed, so most liquid will enter the portal vein. Under normal circumstances, the flow ratio of the portal vein to the hepatic artery in the initial stage of perfusion is 9 : 1. According to the solutions in the prior art, when the perfusion apparatus is in the perfusion mode, a hepatic artery line easily triggers a pressure limit. After the triggering, the hepatic artery does not stop perfusion, but a peristaltic pump stops working, and the perfusion can only be continued by continuously reducing a set flow rate (reducing the pressure). Moreover, during subsequent low-flow perfusion, the hepatic artery is often allocated less than 10% of the flow rate due to the poor internal environment, which, combined with the low overall flow rate, results in less efficient and effective perfusion of the hepatic artery. Therefore, there is an urgent need for a liver perfusion solution to improve the perfusion effect in the event of a poor internal environment of the hepatic artery.

In addition, in the procurement of organs for transplantation, the organs donated by donation after cardiac death (DCD) donors currently account for a large proportion. It has been documented that the oxygen consumption of hepatic tissues decreases with the decrease of the temperature, but does not stop completely at CS (4°C). The range of perfusion oxygenation reported in liver HMP studies lies between 10-100 kPa, but the optimal level of oxygenation is still unknown. Previous studies have confirmed that livers exposed to warm ischemia prior to organ procurement, such as livers of DCD donors, have a higher oxygen demand. The studies have also showed that active oxygenated perfusion had a protective effect on DCD livers compared with non-oxygenated perfusion. In addition, a gas source of a liver machine perfusion apparatus having an oxygenation function on the current market generally comes from an oxygen cylinder, but the oxygen cylinder is inconvenient and dangerous during transportation or use, and cannot be transported by air. Therefore, the liver oxygenated perfusion apparatus on the current market is not portable, and the liver is preserved for a short period of time. Oxygenated machine perfusion during transportation is more beneficial for doctors to organise surgery compared with oxygenated machine perfusion after transportation. Therefore, in addition to addressing the poor internal environments of some hepatic arteries, there is an urgent need for solutions to improve the perfusion effect. In view of a large range of perfusion oxygenation for different livers, there is also an urgent need to develop hardware and control systems for automatic adjustment of the flow and concentration of oxygen to meet different settings of a partial pressure of oxygen in a perfusate. In addition, there is also an urgent need for oxygenated perfusion solutions that can be used continuously in a variety of transportation vehicles.

### Summary of the Invention

The present invention provides a liver hypothermic machine perfusion preservation device, which is particularly suitable for perfusion preservation of a liver with a poor internal environment of a hepatic artery.

The present invention adopts the following technical solutions.

A liver hypothermic perfusion preservation device includes:
a controller configured to control the device; and
a perfusion loop including a main pump, a main reservoir, and a plurality of valve assemblies;
the main pump being in communication with the main reservoir and configured to pump a perfusate from an organ cassette into the main reservoir; and
the main reservoir being in communication with a portal vein and a hepatic artery of a liver respectively by means of a portal vein perfusion line and a hepatic artery perfusion line to supply the perfusate to the portal vein and the hepatic artery,
wherein the perfusion loop further includes an auxiliary reservoir and an auxiliary pump;
the auxiliary reservoir being in communication with the main pump or the main reservoir and configured to obtain the perfusate from the main pump or the main reservoir; and
the auxiliary reservoir being in communication with the hepatic artery by means of a hepatic artery perfusion branch, and the hepatic artery perfusion branch being provided with the auxiliary pump for providing power for the auxiliary reservoir to supply the perfusate to the hepatic artery.

Further, the hepatic artery perfusion branch is in communication with the hepatic artery perfusion line, and the perfusate of the auxiliary reservoir enters the hepatic artery after flowing through the hepatic artery perfusion branch and a part of the hepatic artery perfusion line.

Further, the main reservoir and the auxiliary reservoir are each provided with an exhaust line.

Further, the main reservoir and the auxiliary reservoir are each provided with a liquid level sensor, the main reservoir and the auxiliary reservoir are each provided with a communicating pipe, and the liquid level sensor detects high, medium and low liquid levels in the communicating pipe.

Further, the perfusion loop includes a filter and an oxygenator.

Further, the filter, the oxygenator, the main reservoir and the auxiliary reservoir are jointly placed in a refrigeration area. The refrigeration area is refrigerated by a semiconductor refrigeration element, which is a Peltier refrigeration sheet.

Further, the perfusion loop includes a plurality of bubble sensors, a plurality of flow sensors, a plurality of temperature sensors and/or a plurality of pressure sensors. The plurality of pressure sensors include a total line pressure sensor, a portal vein perfusion line pressure sensor and a hepatic artery perfusion line pressure sensor, and the plurality of pressure sensors are integrated on a circuit board.

Further, the liver hypothermic perfusion preservation device includes a remote operation and display terminal.

Further, the liver hypothermic perfusion preservation device includes an oxygenator and a molecular-sieve oxygen generation unit; the molecular-sieve oxygen generation unit including a compressor, double molecular-sieve towers connected in parallel, a multi-way valve, two gas pressure sensors, a gas flow regulation valve, a gas flow sensor, an oxygen concentration sensor, an oxygen generation line, an air line and a vent line, wherein the oxygen generation line is provided with the double molecular-sieve towers and one of the gas pressure sensors, and the air line is provided with the other of the gas pressure sensors; the oxygen generation line and the air line are connected in parallel to each other and then connected to the vent line, and the vent line is provided with the gas flow regulation valve, the gas flow sensor and the oxygen concentration sensor; and the multi-way valve controls air passing through the compressor to enter at least one of the oxygen generation line and the air line. Further, the oxygen generation line and the air line are each provided with a gas buffer chamber. Further, the portal vein perfusion line is provided with a liquid analysis unit, the liquid analysis unit determining at least a partial pressure of oxygen of the perfusate.

The present invention also provides a liver perfusion method, including:
supplying, by a main pump, a perfusate from a main reservoir to a portal vein and a hepatic artery respectively by means of a portal vein perfusion line and a hepatic artery perfusion line at a flow rate; and
measuring a pressure of the hepatic artery perfusion line, and when the pressure exceeds a set value, and controlling, by a controller, a valve on the hepatic artery perfusion line to be closed to stop the main reservoir from supplying the perfusate to the hepatic artery, while controlling an auxiliary pump to independently supply the perfusate from an auxiliary reservoir to the hepatic artery by means of a hepatic artery perfusion branch.

Further, the perfusate in the auxiliary reservoir is supplied from the main reservoir.

Further, when a liquid level of the perfusate in the auxiliary reservoir is at a low level, the controller controls all valves on the hepatic artery perfusion line, the hepatic artery perfusion branch and the portal vein perfusion line to be closed, and when the liquid level of the perfusate in the auxiliary reservoir rises to a high level, the controller controls the valves on the hepatic artery perfusion branch and the portal vein perfusion line to be opened to restart perfusion of the hepatic artery and the portal vein.

Further, when a liquid level of the perfusate in the main reservoir is at a medium level, the controller controls both valves on the hepatic artery perfusion line and the portal vein perfusion line to be closed, and when the liquid level of the perfusate in the main reservoir rises to a high level, the controller controls the valves on the hepatic artery perfusion line and the portal vein perfusion line to be opened to restart perfusion of the hepatic artery and the portal vein.

Further, the auxiliary pump generates a pulsating flow or a continuous flow.

Further, when a bubble sensor on the hepatic artery perfusion line or on the portal vein perfusion line senses a bubble, valves on the hepatic artery perfusion line and the portal vein perfusion line are controlled to be closed to stop perfusing the hepatic artery and the portal vein, and the perfusate is discharged from an irrigation line in communication with the hepatic artery perfusion line and the portal vein perfusion line.

Further, the method includes an irrigation mode in which the controller controls valves on the hepatic artery perfusion line, the hepatic artery perfusion branch and the portal vein perfusion line to be closed, the perfusate flows into the main reservoir, and when a liquid level of the perfusate in the main reservoir reaches a high level, the irrigation line is opened, and the perfusate is discharged from the irrigation line; and the controller controls the perfusate to flow from the main reservoir into the auxiliary reservoir after the liquid level of the perfusate in the main reservoir is kept at the high level for a period of time, and when a liquid level of the perfusate in the auxiliary reservoir reaches a high level, the irrigation line is opened, and the perfusate is discharged from the irrigation line.

Further, the method includes an exhaust mode in which the hepatic artery of the isolated liver and the hepatic artery perfusion line are connected to two ends of a three-way cannula respectively while the remaining tail end is kept in an open state, and the portal vein of the isolated liver and the portal vein perfusion line are connected to two ends of a further three-way cannula respectively while the remaining tail end is kept in an open state; and the main pump is controlled to run, the perfusate flows through the hepatic artery perfusion line and the portal vein perfusion line, and flows out from the tail ends of the three-way cannulas on the respective lines, to discharge a gas from the lines, and the tail ends of the three-way cannulas are sealed with covers as the perfusate flows out, so as to end the exhaust mode.

Further, the method includes a step of determining a flow rate of the main pump, wherein the controller monitors the time taken by the main reservoir from a low liquid level to a high liquid level as P, a volume of the main reservoir from the low liquid level to the high liquid level is Q, an actual output flow rate of the main pump is calculated as Q/P, and Q/P is compared with a theoretical flow rate value of the main pump to determine whether the flow rate of the main pump is accurate.

Further, the method includes a step of determining a flow rate of the auxiliary pump, wherein the controller monitors the time taken by the auxiliary reservoir from a high liquid level to a low liquid level as P, a volume of the auxiliary reservoir from the low liquid level to the high liquid level is Q, an actual output flow rate of the auxiliary pump is calculated as Q/P, and Q/P is compared with a theoretical flow rate value of the auxiliary pump to determine whether the flow rate of the auxiliary pump is accurate.

The present invention also provides a method for operating a molecular-sieve oxygen generation unit of a liver hypothermic perfusion preservation device, the method including the following steps:
step I of controlling a compressor to run by a controller, wherein air is compressed by the compressor to become compressed air;
step II of controlling a multi-way valve to work by the controller, wherein the compressed air enters a first molecular-sieve tower for nitrogen adsorption, and oxygen flowing out from the first molecular-sieve tower enters an oxygenator through an oxygen generation line and a vent line; and
step III of controlling by the controller, after the first molecular-sieve tower works for a period of time, the multi-way valve to cause the compressed air to enter a second molecular-sieve tower, wherein oxygen flowing out from the molecular-sieve tower is partially supplied to the oxygenator and partially blown back to the first molecular-sieve tower such that nitrogen in the first molecular-sieve tower is discharged from the molecular-sieve oxygen generation unit, and step II is restarted.

Further, a gas flow regulation valve on the vent line automatically regulates an actual flow rate to a target flow rate according to a pressure measured by a gas pressure sensor on the oxygen generation line.

Further, when it is necessary to rapidly reduce a partial pressure of oxygen, the controller controls the multi-way valve to work, and the compressed air enters the oxygenator through an air line and the vent line.

Compared with the prior art, the present invention has the following advantages.
1. In a liver perfusion apparatus in the prior art, a limit pressure of 45 mmHg is usually set for the hepatic artery and the portal vein. When the apparatus is in the perfusion mode, the hepatic artery line easily triggers the pressure limit. The perfusion can only be continued by continuously reducing the set flow rate. Moreover, the hepatic artery is often allocated less than 10% of the flow rate due to the poor internal environment, which results in less efficient and effective perfusion of the hepatic artery. According to the present invention, the auxiliary pump and the auxiliary reservoir are used to provide an additional perfusion flow path for the hepatic artery, so that the perfusion efficiency and effect of the hepatic artery can be improved.
2. The functions of accuracy detection and calibration of the output flow rate of the peristaltic pump are provided.
3. A molecular-sieve oxygen generation function is provided, and a free adjustment range of oxygen concentration from 21% to 90% can be achieved by means of combined control of the molecular-sieve oxygen generation channel and the air channel, thus providing a more flexible solution for the perfusion. During oxygenation of a membrane oxygenator, the oxygenation area of the oxygenator is fixed, and the flow rate of the perfusate is set in direct proportion to the weight of the liver, so that the flow rate of the liquid entering the oxygenator in a single perfusion is also fixed, but the flow rates of the perfusate set for different livers are somewhat different. In a single perfusion, provided that the oxygenation area and the flow rate of the perfusate are determined, the oxygenation efficiency is related to the concentration and the minimum flow rate of oxygen entering the oxygenator, or only related to the concentration of oxygen when the flow rate of oxygen is greater than the minimum flow rate of oxygen. Therefore, the automatic adjustment of the concentration of oxygen is a key factor to balance the set partial pressure of oxygen, and the flow rate of oxygen can be automatically adjusted to be slightly higher than the minimum flow rate. This can further reduce the power consumption of the air compressor. The minimum flow rate of oxygen can be derived from a combination of the magnitude of the liquid flow rate allocated according to different liver weights, the oxygenation area of the oxygenator, and the concentration of oxygen.

### Brief Description of the Drawings

FIG. 1 is a schematic diagram of a first embodiment of the present invention.
FIG. 2 is a schematic control diagram of the first embodiment of the present invention.
FIG. 3 is a schematic diagram of a second embodiment of the present invention.
FIG. 4 is a schematic diagram of a third embodiment of the present invention.
FIG. 5 is a schematic diagram of a fourth embodiment of the present invention.

### Detailed Description of Embodiments

Technical solutions in the embodiments of the present invention will be described below with reference to the accompanying drawings for the embodiments of the present invention. However, the embodiments described below are merely some rather than all of the embodiments of the present invention. On the basis of the embodiments of the present invention, all other embodiments derived by those of ordinary skill in the art without any creative efforts fall within the scope of protection of the present invention.

### Embodiment 1

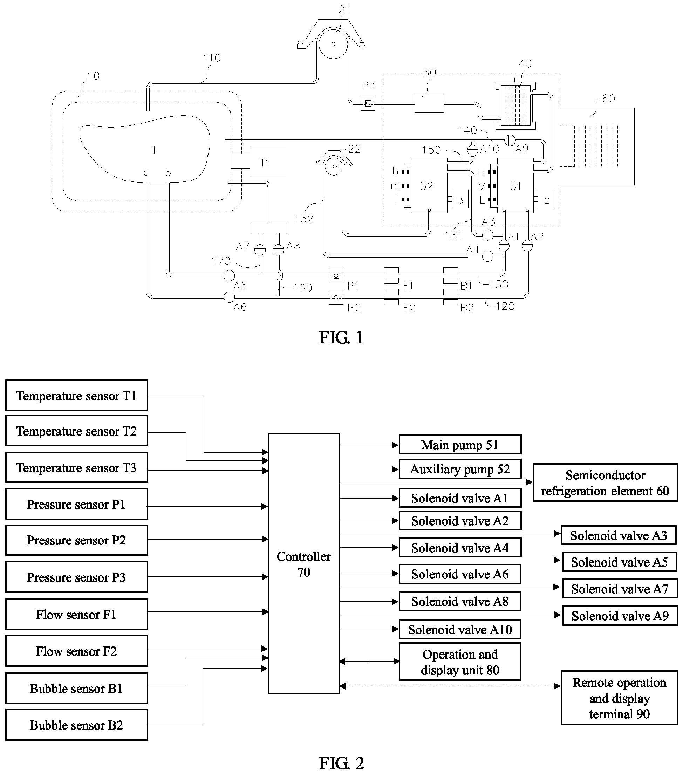

FIGS. 1 and 2 show a first implementation of a liver hypothermic perfusion preservation device, mainly including an organ cassette 10 for placing a liver to be transplanted, an ice box (not shown), a controller 70, an operation and display unit 80, a main pump 21, an auxiliary pump 22, a filter 30, an oxygenator 40, a main reservoir 51, an auxiliary reservoir 52, a semiconductor refrigeration element 60, a sensor assembly and a solenoid valve assembly. The sensor assembly includes bubble sensors B1-B2, pressure sensors P1-P3, flow sensors F1-F2, temperature sensors T1-T3 and liquid level sensors (not shown).

An isolated liver 1 is preserved in the organ cassette 10 containing a perfusate. The organ cassette may be provided with a holder, and the liver is placed on the holder. The organ cassette is arranged in the ice box having a recess, and the ice box may accommodate various cooling media such as ice, ice water and salt water, or any other suitable cooling medium, for example, a polymer phase-change gel material. In use, an organ is placed in the holder, the holder is arranged in the organ cassette, and the organ cassette is arranged in the ice box. In this way, the ice box surrounds the periphery of the organ cassette, so that the organ cassette is kept in a low-temperature environment of 2-8°C. The organ cassette is configured to provide uninterrupted aseptic conditions during transportation, recovery, analysis and storage while maintaining heat transfer with the ice box.

The organ cassette 10, the main pump 21, the filter 30, the oxygenator 40, and the main reservoir 51 are connected to one another in sequence by means of a main line 110.

The main reservoir 51 is connected to a portal vein port a by means of a portal vein perfusion line 120, and the portal vein perfusion line 120 is provided with a solenoid valve A2, a bubble sensor B2, a flow sensor F2, a pressure sensor P2 and a solenoid valve A6 in sequence. The line between the solenoid valve A6 and the pressure sensor P2 is connected to a portal vein perfusion flushing line 160 by means of a three-way valve (not shown), and the portal vein perfusion flushing line 160 is provided with a solenoid valve A8.

The main reservoir 51 is connected to a hepatic artery port b by means of a hepatic artery perfusion line 130, and the hepatic artery perfusion line 130 is provided with a solenoid valve A1, a bubble sensor B1, a flow sensor F1, a pressure sensor P1 and a solenoid valve A5 in sequence. The line between the solenoid valve A5 and the pressure sensor P1 is connected to a hepatic artery perfusion flushing line 170 by means of a three-way valve (not shown), and the hepatic artery perfusion flushing line 170 is provided with a solenoid valve A7.

A temperature sensor T2 and a liquid level sensor are provided in the main reservoir 51. The liquid level sensor can sense whether a liquid level of the perfusate in the main reservoir is at a high level H, a medium level M or a low level L. An upper portion of the main reservoir 51 is connected to the organ cassette by means of a main reservoir exhaust line 140 on which a solenoid valve A9 is arranged.

An inlet of the auxiliary reservoir 52 is connected to the main reservoir 51 by means of an auxiliary reservoir branch 131, an outlet of the auxiliary reservoir 52 is connected to one end of a hepatic artery perfusion branch 132, and the other end of the hepatic artery perfusion branch 132 is connected to the hepatic artery perfusion line 130 by means of a three-way valve (not shown), with a connection point located on the line between the solenoid valve A1 and the bubble sensor B1. In this way, the hepatic artery perfusion flow path of the auxiliary reservoir and the hepatic artery perfusion flow path of the main reservoir can share various sensors, achieving a simple line design. The hepatic artery perfusion branch 132 is provided with the auxiliary pump 22 and a solenoid valve A4.

A temperature sensor T3 and a liquid level sensor are provided in the auxiliary reservoir 52. The liquid level sensor may sense whether a liquid level of the perfusate in the auxiliary reservoir is at a high level h, a medium level m or a low level 1. An upper portion of the auxiliary reservoir 52 is connected to the organ cassette by means of an auxiliary reservoir exhaust line 150 on which a solenoid valve A10 is arranged.

The main pump 21 and the auxiliary pump 22 may be any pump suitable for combination with the perfusion of the organ. Examples of suitable pumps may include hand-operated pumps, centrifugal pumps and peristaltic pumps, and stepper motor peristaltic pumps purchased are used herein.

The filter 30 is configured to filter particles in a liquid path to prevent the particles from entering and blocking the flow path of the device. The particles may be, for example, fat particles or blood clots produced by the isolated organ. The filter may be a single filter or a combination of multiple filters with different apertures, and one 40 µm filter purchased is used herein.

The oxygenator 40 is configured to provide oxygen to the perfusate to increase the oxygen content of the perfusate. Oxygen may be provided to the oxygenator by means of any suitable device. A suitable oxygen source may be hospital high-pressure oxygen, a large oxygen cylinder or a customised small oxygen cylinder.

The main reservoir 51 and the auxiliary reservoir 52 are configured to temporarily store the perfusate in the flow path and act as a bubble trap to separate bubbles that may be entrained in the perfusate flow, so as to prevent these bubbles from continuing downstream and entering the isolated organ.

The bubble sensors B1-B2 may be ultrasonic bubble sensors. The ultrasonic bubble sensors may not be in contact with the perfusate, and thus do not need to be cleaned and are easy to replace.

The flow sensors F1-F2 are configured to detect the flow rate of the perfusate flowing through the lines, and the flow sensors may be ultrasonic flow sensors.

The pressure sensors P1-P3 measure real-time pressures of the liquid paths and provide over-pressure monitoring. The pressure sensor measures pressures at two points and obtains a pressure value by means of averaging. The pressure sensor P3 connected to the main pump measures the total pressure of the lines, and the pressure sensors P1 and P2 respectively measure the pressures of the hepatic artery perfusion line and the portal vein perfusion line. By comparing the pressures at the two points that are measured by the pressure sensor, or by comparing the total pressure with the pressure values of the hepatic artery perfusion line and the portal vein perfusion line, the accuracy of the pressure sensor can be effectively indicated. If the difference between the pressures at the two points of the perfusion line is too large, the device may indicate an error. If the sum of the pressures of the hepatic artery perfusion line and the portal vein perfusion line is significantly different from the total pressure, the device may also indicate an error. In this embodiment, the pressure sensors P1, P2 and P3 are independently arranged on their respective lines, or all the pressure sensors may be integrated on a circuit board.

The liquid level sensor is in the form of a communicating pipe, and a side surface of each of the main reservoir and the auxiliary reservoir is provided with a communicating pipe. The communicating pipe is a rigid semi-mouth-shaped pipe and is mounted on an outer wall of the reservoir, and the bottom and the top of the outer wall of the reservoir are provided with line interfaces and are connected to the communicating pipe. Three photoelectric sensors are mounted on a vertical hard pipe wall of the communicating pipe to detect the low, medium and high liquid levels. When the reservoir is in communication with the atmosphere, the liquid enters the reservoir and the communicating pipe, and the liquid level of the communicating pipe is kept consistent with that of the reservoir. By detecting the liquid in the communicating pipe, the liquid level of the reservoir is determined and the output flow rate of the peristaltic pump is detected. When the reservoir is not in communication with the atmosphere, the reservoir is in a closed state, the peristaltic pump pumps the liquid into the reservoir, and pressure is generated in the reservoir to press the liquid into the line. The air portion of the reservoir and the air portion of the communicating pipe are connected to each other and are under the same air pressure, so the same liquid level can be maintained.

The operation and display unit 80 is provided with an operation input portion and a display unit. The operation input portion provides functions of setting the temperature, the pressure, the output flow rate of the pump, etc. The display unit enables the whole process display of perfusion parameters, and display of device running modes, running progress, etc.

In this embodiment, the filter 30, the oxygenator 40, the main reservoir 51 and the auxiliary reservoir 52 are placed in the same refrigeration area, with a refrigerator being used herein. The refrigerator takes a semiconductor refrigeration element 60 as a refrigeration source. The semiconductor refrigeration element is a Peltier refrigeration sheet assembly. The organ cassette 10, the main reservoir 51 and the auxiliary reservoir 52 are respectively provided with three temperature sensors T1, T2, T3, which respectively measure the temperature of the perfusate in the organ cassette, the temperature of the perfusate in the main reservoir and the temperature of the perfusate in the auxiliary reservoir. When any temperature exceeds a set value of 8°C, the controller 70 controls the Peltier refrigeration sheet assembly to start so as to reduce the temperature of the perfusate to the set value. Such a design can effectively ensure the temperature requirements of the perfusate during long-time perfusion or when a sealing cover is frequently opened due to clinical needs.

In this embodiment, the device includes an irrigation mode, an exhaust mode and a perfusion mode.

### Irrigation mode

In a power-on irrigation mode, the controller 70 controls the main pump to provide a uniform flow rate of 2 L/min. The controller 70 controls the solenoid valves A1, A2, A3, A4, A5, A6, A7, A8, A10 to be closed and the solenoid valve A9 to be opened. The perfusate from the organ cassette passes through the main pump 21, the filter 30 and the oxygenator 40 to reach the main reservoir 51, and the gas in the main reservoir is discharged from the line system through the main reservoir exhaust line 140.

The liquid level sensor detects the liquid level of the perfusate in the main reservoir. When the liquid level reaches the high level H, the controller controls the solenoid valves A3, A4, A5, A6, A9, A10 to be closed, and the solenoid valves A1, A2, A7, A8 to be opened, and the perfusate returns to an organ chamber through the bubble sensors B1-B2, the flow sensors F1-F2, the pressure sensors P1-P2 and the irrigation lines 160 and 170.

After keeping the perfusate in the main reservoir at the high level for 2 min, the controller controls the solenoid valves A1, A2, A4, A5, A6, A7, A8, A9 to be closed, and the solenoid valves A3, A10 to be opened, so that the perfusate enters the auxiliary reservoir 52 from the main reservoir 51, and the gas in the auxiliary reservoir is discharged from the line system through the exhaust line 150.

The liquid level sensor detects the liquid level of the perfusate in the auxiliary reservoir 52. When the liquid level reaches the high level h, the controller controls the solenoid valves A1, A3, A5, A6, A9 to be closed, and the solenoid valves A2, A4, A7, A8, A10 to be opened. The controller controls the auxiliary pump 22 to run at a constant speed of 150 ml/min. The perfusate is pumped out from the auxiliary reservoir 52, is merged into the hepatic artery perfusion line 130 through the hepatic artery perfusion branch 132, and returns to the organ chamber through the bubble sensor B1, the flow sensor F1, the pressure sensor P1, and the irrigation line 170.

When the liquid level of the perfusate in the auxiliary reservoir 52 reaches the low level 1, the controller controls the auxiliary pump 22 to stop running, the solenoid valves A3, A4, A5, A6, A9, A10 to be closed, and the solenoid valves A1, A2, A7, A8 to be opened, and the irrigation mode ends after another 2 min.

### Exhaust mode

The hepatic artery of the isolated liver and the hepatic artery perfusion line 130 of the device are connected to two ends of a three-way cannula respectively while the remaining tail end is kept in an open state. The portal vein of the isolated liver and the portal vein perfusion line 120 of the device are connected to two ends of a further three-way cannula respectively while the remaining tail end is kept in an open state.

The main pump is controlled to run at a uniform flow rate of 0.3 L/min. The solenoid valves A1, A2, A5 and A6 are controlled to be opened and the solenoid valves A3, A4, A7, A8, A9, A10 are controlled to be closed. The perfusate flows through the hepatic artery perfusion line 130 and the portal vein perfusion line 120, and flows out from the tail ends of the three-way cannulas on their respective lines, to discharge the gas from the lines. A part of the perfusate may enter blood vessels of the liver connected to the three-way cannula after the perfusate enters the three-way cannula. The gas in the blood vessels can be discharged by adjusting the angle of the three-way cannula.

The tail end of the three-way cannula is sealed with a cover when the liquid flows out, so as to end the exhaust mode.

### Perfusion mode

The controller controls the main pump 21 to run at a uniform speed according to a set flow rate. The flow rate is set according to the weight of the liver, and the set flow rate per minute is calculated based on 0.66 L/kg, and the system may automatically generate the set flow rate after the weight of the liver is input.

The solenoid valves A1, A2, A5, A6 are controlled to be opened and the solenoid valves A3, A4, A7, A8, A9, A10 are controlled to be closed. The perfusate of the organ cassette 10 enters the main reservoir 51 through the filter 30 and the oxygenator 40, the main reservoir stores the perfusate and the line pressure, the perfusate is pressed into the hepatic artery perfusion line 130 and the portal vein perfusion line 120, and the perfusate finally enters the hepatic artery and the portal vein, thus realizing freely-distributed perfusion.

When the pressure sensor P1 detects that the line pressure of the hepatic artery reaches the set value of 45 mmHg, the controller controls the solenoid valves A1, A2, A4, A5, A6, A7, A8, A9 to be closed, and the solenoid valves A3, A10 to be opened, so that the perfusate enters the auxiliary reservoir 52 from the main reservoir 51. When the liquid level of the auxiliary reservoir 52 reaches the high level h, the solenoid valves A1, A3, A7, A8, A9 are closed, while the solenoid valves A2, A4, A5, A6, A10 are opened, and the auxiliary pump 22 starts to perform pulsating or continuous independent perfusion on the hepatic artery at a fixed pressure of 40 mmHg. The advantage of fixed-pressure perfusion is that the auxiliary pump may keep the set perfusion pressure unchanged in the whole process of perfusion, so that perfusion will not stop due to excessive resistance of the hepatic artery. Moreover, the fixed-pressure perfusion mode is a safe perfusion mode. In case of excessive resistance of the hepatic artery, it is easier to reach the set pressure and the line flow is low. With continuous perfusion at a low flow rate, the resistance decreases and the line flow increases, so that the fixed-pressure perfusion can effectively avoid possible machine damages to the liver during perfusion.

Moreover, the controller controls the main pump to reduce the flow rate by 25%. In this case, the main reservoir 51 is only in communication with the portal vein and only the portal vein is perfused. When the liquid level of the auxiliary reservoir 42 reaches the low level 1, the solenoid valves A1, A2, A4, A5, A6, A7, A8, A9 are closed, and the solenoid valves A3, A10 are opened. The auxiliary reservoir 52 continues the independent perfusion after replenishment.

When the line flow of the hepatic artery reaches 15% or more of an initially set flow rate of the main pump, i.e., the hepatic artery has basically been perfused smoothly, the auxiliary pump continues to run until the auxiliary reservoir reaches the low liquid level, and the controller controls the auxiliary pump to stop running, and controls the solenoid valves A1, A2, A5, A6 to be opened, and the solenoid valves A3, A4, A7, A8, A9, A10 to be closed, so that the perfusion of the hepatic artery and the portal vein can be implemented again by the main reservoir.

When any bubble sensor senses a bubble, the controller controls the solenoid valves A5, A6 to be closed, and the solenoid valves A7, A8 to be opened, and is switched back to the perfusion mode after irrigation for 1 min.

During perfusion, if the liquid level of the main reservoir is lower than the medium liquid level M, the controller controls the solenoid valves A1, A2 to be closed, and the solenoid valve A9 to be opened, and is switched back to the perfusion mode after the high liquid level is reached by replenishment.

When the auxiliary pump performs independent perfusion on the hepatic artery, the total pressure on a pressure sensor module is only related to the portal vein, and the pressure of the hepatic artery is monitored independently and is not related to the total pressure.

The device can also enable automatic detection and calibration of the output flow rate of the main pump, and as set by the controller, the main pump rotates at the highest constant speed of rotation. The theoretical maximum output flow is LM (ml/min) if the theoretical flow rate of the peristaltic pump is L per revolution and the number of revolutions per minute is M. The controller may monitor the time taken by the main reservoir from the low liquid level to the high liquid level, for example, P min, and the volume of the main reservoir from the low liquid level to the high liquid level is also known, for example, Q ml. Then, the actual output flow rate can be calculated as Q/P (ml/min). The actual value Q/P is compared with the theoretical value LM to determine whether the flow rate of the main pump is accurate, and the output flow rate of the main pump can be calibrated according to this method. A method for determining the output flow rate of the auxiliary pump is basically the same as that of the main pump, and an actual output flow parameter of the auxiliary pump can be obtained by calculating the time taken by the auxiliary reservoir from the high liquid level to the low liquid level. By comparing and analysing the actual output flow parameters, the theoretical output flow parameters and the flow sensor detection parameters of the main pump and the auxiliary pump, the accuracy of the flow rate of the liquid path can be prompted and alarmed.

The device is connected to the remote operation and display terminal 90 through the Internet of Things, thus can remotely enable the whole process display of perfusion parameters, the display of running mode and progress of the device, and the automatic storage of perfusion reports, and can be remotely controlled.

### Embodiment 2

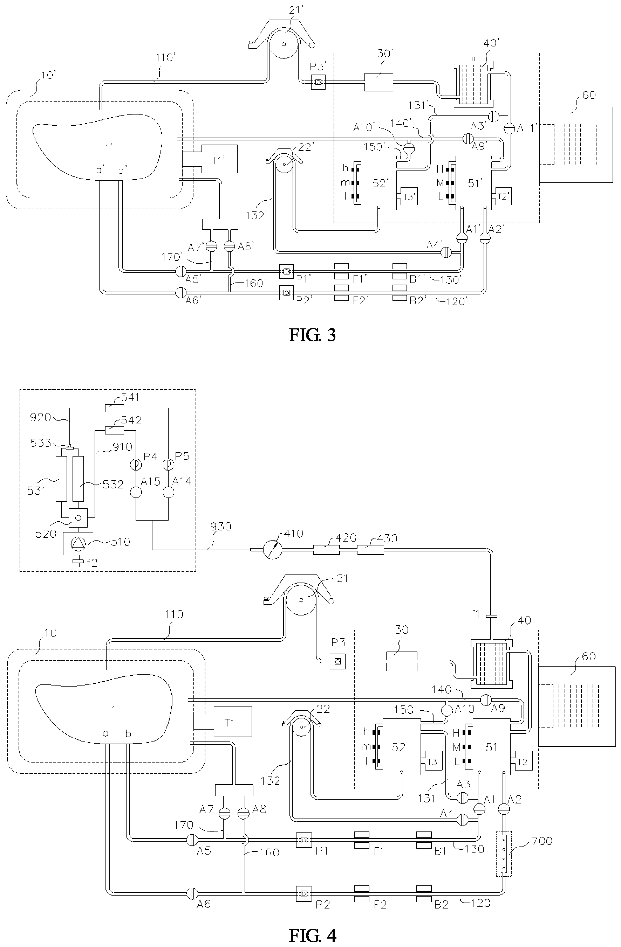

FIG. 3 shows a second implementation of a liver hypothermic perfusion preservation device, mainly including an organ cassette 10', an ice box (not shown), a controller (not shown), an operation and display unit (not shown), a main pump 21', an auxiliary pump 22', a filter 30', an oxygenator 40', a main reservoir 51', an auxiliary reservoir 52', a semiconductor refrigeration element 60', a sensor assembly and a solenoid valve assembly. The sensor assembly includes bubble sensors B1'-B2', pressure sensors P1'-P3', flow sensors F1'-F2', temperature sensors T1'-T3' and liquid level sensors (not shown).

The organ cassette 10', the main pump 21', the filter 30' and the oxygenator 40' are connected to one another in sequence by means of a main line 110'.

An outlet of the oxygenator 40' is connected to both a main reservoir branch 121 and an auxiliary reservoir branch 131' by means of a three-way valve (not shown).

The main reservoir branch 121 is connected to the main reservoir 51'. The main reservoir branch 121 is provided with a solenoid valve A11. The main reservoir 51' is connected to a portal vein port a' by means of a portal vein perfusion line 120', and the portal vein perfusion line 120' is provided with a solenoid valve A2', a bubble sensor B2', a flow sensor F2', a pressure sensor P2' and a solenoid valve A6' in sequence. The line between the solenoid valve A6' and the pressure sensor P2' is connected to a portal vein perfusion flushing line 160' by means of a three-way valve (not shown), and the portal vein perfusion flushing line 160' is provided with a solenoid valve A8'.

The main reservoir 51' is connected to a hepatic artery port b' by means of a hepatic artery perfusion line 130', and the hepatic artery perfusion line 130' is provided with a solenoid valve A1', a bubble sensor B1', a flow sensor F1', a pressure sensor P1' and a solenoid valve A5' in sequence. The line between the solenoid valve A5' and the pressure sensor P1' is connected to a hepatic artery perfusion flushing line 170' by means of a three-way valve (not shown), and the hepatic artery perfusion flushing line 170' is provided with a solenoid valve A7'.

A temperature sensor T2' and a liquid level sensor are provided in the main reservoir 51'. The liquid level sensor can sense whether a liquid level of the perfusate in the main reservoir is at a high level H, a medium level M or a low level L. An upper portion of the main reservoir 51' is connected to the organ cassette by means of a main reservoir exhaust line 140' on which a solenoid valve A9' is arranged.

The oxygenator 40' is connected to an inlet of the auxiliary reservoir 52' by means of an auxiliary reservoir branch 131'. The auxiliary reservoir branch 131' is provided with a solenoid valve A3'. An outlet of the auxiliary reservoir 52' is connected to one end of a hepatic artery perfusion branch 132', and the other end of the hepatic artery perfusion branch 132' is connected to the hepatic artery perfusion line 130' by means of a three-way valve (not shown), with a connection point located on the line between the solenoid valve A1' and the bubble sensor B1'. The hepatic artery perfusion branch 132' is provided with the auxiliary pump 22' and a solenoid valve A4'.

A temperature sensor T3' and a liquid level sensor are provided in the auxiliary reservoir 52'. The liquid level sensor may sense whether a liquid level of the perfusate in the auxiliary reservoir is at a high level h, a medium level m or a low level 1. An upper portion of the auxiliary reservoir 52' is connected to the organ cassette by means of an auxiliary reservoir exhaust line 150' on which a solenoid valve A10' is arranged.

Similar to Embodiment 1, in this embodiment, the filter 30', the oxygenator 40', the main reservoir 51' and the auxiliary reservoir 52' are placed in the same refrigerator, and the refrigerator takes a semiconductor refrigeration element 60' as a refrigeration source.

In this embodiment, the device includes an irrigation mode, an exhaust mode and a perfusion mode.

### Irrigation mode

In a power-on irrigation mode, the controller controls the main pump to provide a uniform flow rate of 2 L/min. The controller controls the solenoid valves A1', A2', A3', A4', A5', A6', A7', A8', A10' to be closed, and the solenoid valves A9' and A11 to be opened. The perfusate from the organ cassette passes through the main pump 21', the filter 30' and the oxygenator 40' to reach the main reservoir 51', and the gas in the main reservoir is discharged from the line system through the main reservoir exhaust line 140'.

The liquid level sensor detects the liquid level of the perfusate in the main reservoir. When the liquid level reaches the high level H, the controller controls the solenoid valves A1', A2', A4', A5', A6', A7', A8', A9', A11 to be closed, and the solenoid valves A3', A10' to be opened. The perfusate from the organ cassette passes through the main pump 21', the filter 30' and the oxygenator 40' to reach the auxiliary reservoir 52', and the gas in the auxiliary reservoir is discharged from the line system through the auxiliary reservoir exhaust line 150'.

The liquid level sensor detects the liquid level of the perfusate in the auxiliary reservoir. When the liquid level reaches the high level H, the controller controls the solenoid valves A3', A5', A6', A9' to be closed, the solenoid valves A1', A2', A4', A7', A8', A10', A11 to be opened, and the auxiliary pump starts to run at a constant speed of 150 ml/min.

The perfusate in the main reservoir enters the portal vein perfusion line 120', and returns to an organ chamber from the irrigation line 160' through the bubble sensor B2', the flow sensor F2' and the pressure sensor P2'. The perfusate in the main reservoir enters the hepatic artery perfusion line 130', and returns to the organ chamber from the irrigation line 170' through the bubble sensor B1', the flow sensor F1' and the pressure sensor P1'. The perfusate in the auxiliary reservoir also flows into the hepatic artery perfusion line 130' through the hepatic artery perfusion branch 132'. When the liquid in the auxiliary reservoir reaches a low level, the auxiliary pump stops running, and the controller controls the solenoid valves A4' and A10' to be closed, and the irrigation mode ends after another 2 min.

### Exhaust mode

The hepatic artery of the isolated liver and the hepatic artery perfusion line 130' of the device are connected to two ends of a three-way cannula respectively while the remaining tail end is kept in an open state. The portal vein of the isolated liver and the portal vein perfusion line 120' of the device are connected to two ends of a further three-way cannula respectively while the remaining tail end is kept in an open state.

The main pump is controlled to run at a uniform flow rate of 0.3 L/min. The solenoid valves A1', A2', A5', A6', A11 are controlled to be opened, and the solenoid valves A3', A4', A7', A8', A9', A10' are controlled to be closed. The perfusate flows through the hepatic artery perfusion line 130' and the portal vein perfusion line 120', and flows out from the tail ends of the three-way cannulas on their respective lines, to discharge the gas from the lines. A part of the perfusate may enter blood vessels of the liver connected to the three-way cannula after the perfusate enters the three-way cannula. The gas in the blood vessels can be discharged by adjusting the angle of the three-way cannula.

The tail end of the three-way cannula is sealed with a cover when the liquid flows out, so as to end the exhaust mode.

### Perfusion mode

The controller controls the main pump 21' to run at a uniform speed according to a set flow rate. The solenoid valves A1', A2', A5', A6', A11 are controlled to be opened, and the solenoid valves A3', A4', A7', A8', A9', A10' are controlled to be closed. The perfusate enters the hepatic artery and the portal vein through the hepatic artery perfusion line 130' and the portal vein perfusion line 120' respectively, thus realizing freely-distributed perfusion.

When the pressure sensor P1' detects that the line pressure of the hepatic artery reaches a set value of 45 mmHg, the controller controls the solenoid valves A4', A7', A8', A9' to be closed, and the solenoid valves A1', A2', A3', A5', A6', A10', A11 to be opened. Most of the perfusate enters the auxiliary reservoir by means of the solenoid valve A3'. When the liquid level in the auxiliary reservoir reaches a high level, the solenoid valves A1', A3', A7', A8', A9' are controlled to be closed, and the solenoid valves A2', A4', A5', A6', A10', A11 are controlled to be opened. Moreover, the main pump only perfuses the portal vein after automatic reduction of the flow rate by 25%, and the auxiliary pump perfuses the hepatic artery with a fixed pressure of 40 mmHg.

When the liquid level of the auxiliary reservoir reaches a low level, the solenoid valves A1', A4', A7', A8', A9' are controlled to be closed, and the solenoid valves A2', A3', A5', A6', A10', A11 are controlled to be opened. The flow rate of the main pump is unchanged, and after the auxiliary reservoir is replenished to reach the high liquid level, the solenoid valves A1', A3', A7', A8', A9' are controlled to be closed, and the solenoid valves A2', A4', A5', A6', A10', A11 are controlled to be opened. The previous step of perfusion is continued and the cycle is repeated.

When the line flow of the hepatic artery reaches 15% or more of an initially set flow rate of the main pump, the auxiliary pump continues to run until the auxiliary reservoir reaches the low liquid level, and the controller controls the auxiliary pump to stop running, and controls the solenoid valves A1', A2', A5', A6', A11 to be opened, and the solenoid valves A3', A4', A7', A8', A9', A10' to be closed, so that the perfusion of the hepatic artery and the portal vein can be implemented again by the main reservoir.

When any bubble sensor senses a bubble, the controller controls the solenoid valves A5', A6' to be closed, and the solenoid valves A7', A8' to be opened, and is switched back to the perfusion mode after irrigation for 1 min.

During perfusion, if the liquid level of the main reservoir is lower than the medium liquid level, the controller controls the solenoid valves A1', A2' to be closed, and the solenoid valve A9' to be opened, and is switched back to the perfusion mode after the high liquid level is reached by replenishment.

### Embodiment 3

FIG. 4 shows a third implementation of a liver hypothermic perfusion preservation device. In this embodiment, based on Embodiment 1, a molecular-sieve oxygen generation unit is used as an oxygen source of the oxygenator, and perfusion units use the same numbers as in Embodiment 1.

The molecular-sieve oxygen generation unit includes a filter f1, a filter f2, a compressor 510, a five-way valve 520, a molecular-sieve tower 531, a molecular-sieve tower 532, a T-joint 533, a gas buffer chamber 541, a gas buffer chamber 542, a gas pressure sensor P4, a gas pressure sensor P5, a gas flow regulation valve 410, a gas flow sensor 420, an oxygen concentration sensor 430, an air line 910, an oxygen generation line 920 and a vent line 930.

Outside air enters the compressor 510 from the filter f2 and then is introduced into the molecular-sieve tower 531 or the molecular-sieve tower 532 or the air line 910 by means of the five-way valve 520. The five-way valve 520 may control the compressed air to enter the molecular-sieve tower 531 or the molecular-sieve tower 532 or the air line 910 alone, or may control the compressed air to enter any two or three of the molecular-sieve tower 531, the molecular-sieve tower 532 and the air line 910. The five-way valve 520 also has an exhaust port capable of releasing pressure in the molecular-sieve tower 531, the molecular-sieve tower 532 and the air line 910. Gases flowing out from the molecular-sieve tower 531 and the molecular-sieve tower 532 are merged into the oxygen generation line 920 by means of the T-joint 533. The oxygen generation line 920 is provided with the gas buffer chamber 541, the gas pressure sensor P5 and a solenoid valve A14 in sequence. The air line 910 is provided with the gas buffer chamber 542, the gas pressure sensor P4 and a solenoid valve A15 in sequence. Gases flowing out from the air line 910 and the oxygen generation line 920 are merged into the vent line 930. The vent line 930 is provided with the gas flow regulation valve 410, the gas flow sensor 420, the oxygen concentration sensor 430 and the filter f1 in sequence, and the gas flowing out from the vent line 930 enters the oxygenator 40.

The molecular-sieve tower separates nitrogen in air by using the molecular sieve technology to obtain high-concentration oxygen. Specifically, high-pressure air is forced into the molecular-sieve tower by using the compressor, and nitrogen molecules in air are adsorbed by a molecular sieve to obtain high-purity oxygen. The molecular-sieve tower uses a zeolite molecular sieve as an adsorbent, and by utilizing the characteristic that the zeolite molecular sieve full of micropores has different adsorption capacities for oxygen and nitrogen in air, preferably adsorbs harmful gases such as carbon dioxide, sulfide and nitrogen in air, so as to obtain high-purity oxygen that meets medical standards and is suitable for being carried by the perfusion system. For example, a 5A zeolite molecular sieve may be used as the molecular sieve in the present invention. The 5A zeolite has a structural formula of 3/4CaO·1/4Na₂O·Al₂O₃·2SiO₂.9/2H₂O, and an aperture of 5A, and can adsorb any molecule having a smaller aperture. The 5A zeolite is selected for its high adsorption capacity and high adsorption rate. Molecular-sieve zeolite is sealed in a tower by means of physical packaging after filling, and the amount of filling is generally controlled to be 90% of the tower cavity.

The gas flow regulation valve may use a stepper-motor-driven connecting rod for fine adjustment of an effective diameter of the gas channel in order to implement gas flow regulation, and can specifically use a conical-head connecting rod, a cylindrical connecting rod and the like. Flow control may also be implemented by using a gas flow regulation valve such as a solenoid valve.

The compressor 510, the five-way valve 520, the pressure sensor P4, the pressure sensor P5, the solenoid valve A14, the solenoid valve A15, the gas flow regulation valve 410, the gas flow sensor 420, and the oxygen concentration sensor 430 are all connected to the controller 70, and can perform input operations on the operation and display unit 80.

### Oxygen generation mode

The target oxygen concentration, the flow rate of oxygen, and a fixed value or a range of a partial pressure of oxygen are set on the operation and display unit 80. The compressor 510 starts to work, and the five-way valve 520 controls the compressed air to enter the molecular-sieve tower 531. The pressure of the molecular-sieve tower 531 rises, and the zeolite filler may adsorb nitrogen to increase the oxygen concentration. After a fixed period of time, the five-way valve 520 controls the compressed air to also enter the molecular-sieve tower 532, and an intake port of the molecular sieve 531 is closed after a brief period of simultaneous intake. The intake port of the molecular sieve 531 is controlled to be in communication with the exhaust port of the five-way valve 520. After the pressure of the molecular-sieve tower 531 is released, nitrogen may be separated and pushed back by oxygen of the T-joint 533 so as to be discharged through the exhaust port of the five-way valve 520. Then, the exhaust port of the five-way valve 520 is closed and the molecular-sieve tower 532 continues to generate oxygen. After working for a period of time, the molecular-sieve tower 532 is replaced with the molecular-sieve tower 531. This cycle can be repeated for continuous oxygen generation.

The gas pressure sensor P5 measures the internal pressure from the molecular-sieve tower to the gas flow regulation valve 410. The gas flow regulation valve 410 automatically adjusts, according to the different internal pressures, the connecting rod to change the effective diameter of the gas channel so as to achieve the target flow rate. Moreover, the stable internal pressure is related to the oxygen concentration. While achieving the target flow rate, the controller 70 automatically adjusts the power of the compressor 510 so as to achieve the related stable internal pressure and therefore the set oxygen concentration. That is, the target flow rate of oxygen and the target oxygen concentration are achieved in such a way that the controller 70 controls the power of the compressor by means of a PID algorithm, based on the fact that the oxygen concentration is linearly related to the internal pressure, the internal pressure is linearly related to the duty ratio of the compressor, and the flow rates at different pressures are linearly related to the angle of rotation of the connecting rod of a gas flow regulator.

### Perfusion mode

In the oxygenated machine perfusion mode, a line 120 connected to the main reservoir 51 is provided with a liquid analyser 700. The liquid analyser is located downstream of the solenoid valve A2 on the line 120 and connected to the controller 70. The liquid analyser 700 may be configured as a consumable integrated with a disposable perfusion line to analyse a partial pressure of oxygen, a partial pressure of carbon dioxide, a pH value and lactic acid in the perfusate. The partial pressure of oxygen, the partial pressure of carbon dioxide, the pH value and the lactic acid detected by the liquid analyser 700 can guide the perfusion process, and the range or the fixed value of the partial pressure of oxygen may be set in the operation and display unit 80 according to different organs and perfusion conditions. The controller 70 may automatically adjust the concentration and the flow rate of oxygen entering the oxygenator from the molecular-sieve oxygen generation unit, or intermittently start the gas source to automatically achieve a set target partial pressure of oxygen.

The difference from a conventional molecular-sieve oxygen generator is that the five-way valve 520 also has an interface such that the filtered compressed air can be directly introduced into the gas buffer chamber 542. The gas buffer chamber 542 is connected to the independent pressure sensor P4 and solenoid valve A15. The concentration of oxygen in air is 21%. When the partial pressure of oxygen needs to be quickly reduced during perfusion, air can be introduced into the oxygenator before the gas source is cut off. During perfusion, different perfusates have different dissolution rates of oxygen. A free adjustment range of oxygen concentration from 21% to 90% can be achieved by means of combined control of an air channel and a molecular-sieve oxygen generation channel, thus providing a more flexible solution for the perfusion. Moreover, if a special oxygenator is used, introducing air into the oxygenator can also adjust the partial pressure of carbon dioxide in the perfusate.

### Embodiment 4

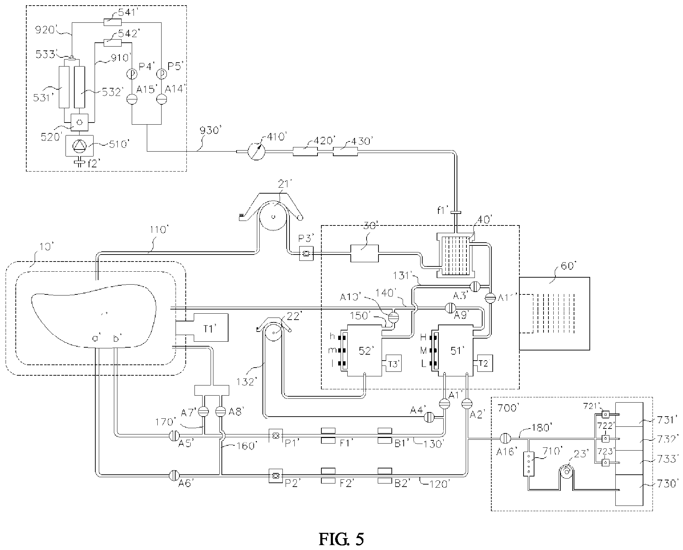

FIG. 5 shows a fourth implementation of a liver hypothermic perfusion preservation device. In this embodiment, based on Embodiment 2, a molecular-sieve oxygen generation unit is used as an oxygen source of the oxygenator, and perfusion units use the same numbers as in Embodiment 2.

The molecular-sieve oxygen generation unit includes a filter f1', a filter f2', a compressor 510', a five-way valve 520', a molecular-sieve tower 531', a molecular-sieve tower 532', a T-joint 533', a gas buffer chamber 541', a gas buffer chamber 542', a gas pressure sensor P4', a gas pressure sensor P5', a gas flow regulation valve 410', a gas flow sensor 420', an oxygen concentration sensor 430', an air line 910', an oxygen generation line 920' and a vent line 930'. The connection and working modes of the above components are the same as those in Embodiment 3.

In this embodiment, the portal vein perfusion line 120' is provided with a liquid analysis unit 700', and the liquid analysis unit 700' is located downstream of the solenoid valve A2'. The liquid analysis unit 700' includes a line 180'. The line 180' is controlled by a solenoid valve A16'. A liquid analysis electrode 710', an analysis pump 23', an analysis reagent and corresponding control valves, and a waste liquid tank are arranged at a rear end of the solenoid valve A16'. When liquid test is performed, the valve A16' is closed, and the analysis pump 23' rotates to pump a test reagent to calibrate the analysis electrode. Then, the main pump stops running. The solenoid valves A11', A6', A8' are closed, and the solenoid valves A9', A16' are opened. The analysis pump 23' rotates to suck in an amount of perfusate into an electrode plate for liquid analysis. Waste liquid is pumped into a waste liquid bottle and no longer enters the perfusion line. The liquid analyser 700', the electrode plate 710' in the liquid analysis unit 700', the analysis pump 23', a reagent valve 721', a reagent valve 722' and a reagent valve 723' are all connected to the controller 70'.

The partial pressure of oxygen, the partial pressure of carbon dioxide, the pH value and the lactic acid detected by the liquid analysis unit 700' can guide the perfusion process, and the range or the fixed value of the partial pressure of oxygen may be set in the operation and display unit 80' according to different organs and perfusion conditions. The controller 70' may automatically adjust the concentration and the flow rate of oxygen entering the oxygenator from the molecular-sieve oxygen generation unit, or intermittently start the gas source to automatically achieve a set target partial pressure of oxygen.

## Claims

1. A liver hypothermic perfusion preservation device, comprising:
a controller configured to control the device; and
a perfusion loop comprising a main pump, a main reservoir, and a plurality of valve assemblies;
the main pump being in communication with the main reservoir and configured to pump a perfusate from an organ cassette into the main reservoir; and
the main reservoir being in communication with a portal vein and a hepatic artery of a liver respectively by means of a portal vein perfusion line and a hepatic artery perfusion line to supply the perfusate to the portal vein and the hepatic artery,
**characterised in that** the perfusion loop further comprises an auxiliary reservoir and an auxiliary pump;
the auxiliary reservoir being in communication with the main pump or the main reservoir and configured to obtain the perfusate from the main pump or the main reservoir; and
the auxiliary reservoir being in communication with the hepatic artery by means of a hepatic artery perfusion branch, and the hepatic artery perfusion branch being provided with the auxiliary pump for providing power for the auxiliary reservoir to supply the perfusate to the hepatic artery.

2. The liver hypothermic perfusion preservation device according to claim 1, **characterised in that** the hepatic artery perfusion branch is in communication with the hepatic artery perfusion line.

3. The liver hypothermic perfusion preservation device according to claim 1, **characterised in that** the main reservoir and the auxiliary reservoir are each provided with an exhaust line.

4. The liver hypothermic perfusion preservation device according to claim 1, **characterised in that** the main reservoir and the auxiliary reservoir are each provided with a liquid level sensor.

5. The liver hypothermic perfusion preservation device according to claim 4, **characterised in that** the main reservoir and the auxiliary reservoir are each provided with a communicating pipe, and the liquid level sensor detects high, medium and low liquid levels of the perfusate in the communicating pipe.

6. The liver hypothermic perfusion running device according to claim 1, **characterised in that** the perfusion loop comprises a filter and an oxygenator.

7. The liver hypothermic perfusion preservation device according to claim 6, **characterised in that** the filter, the oxygenator, the main reservoir and the auxiliary reservoir are jointly placed in a refrigeration area.

8. The liver hypothermic perfusion preservation device according to claim 7, **characterised in that** the refrigeration area is refrigerated by a semiconductor refrigeration element.

9. The liver hypothermic perfusion preservation device according to claim 8, **characterised in that** the semiconductor refrigeration element is a Peltier refrigeration sheet.

10. The liver hypothermic perfusion preservation device according to claim 1, **characterised in that** the perfusion loop comprises a plurality of bubble sensors, a plurality of flow sensors, a plurality of temperature sensors and/or a plurality of pressure sensors.

11. The liver hypothermic perfusion preservation device according to claim 10, **characterised in that** the plurality of pressure sensors comprise a total line pressure sensor, a portal vein perfusion line pressure sensor and a hepatic artery perfusion line pressure sensor.

12. The liver hypothermic perfusion preservation device according to either of claims 10-11, **characterised in that** the plurality of pressure sensors are integrated on a circuit board.

13. The liver hypothermic perfusion preservation device according to claim 1, **characterised by** further comprising a remote operation and display terminal.

14. The liver hypothermic perfusion preservation device according to claim 1, **characterised by** further comprising an oxygenator and a molecular-sieve oxygen generation unit, the molecular-sieve oxygen generation unit providing oxygen for the oxygenator; and
the molecular-sieve oxygen generation unit comprising a compressor, double molecular-sieve towers connected in parallel, a multi-way valve, two gas pressure sensors, a gas flow regulation valve, a gas flow sensor, an oxygen concentration sensor, an oxygen generation line, an air line and a vent line, wherein the oxygen generation line is provided with the double molecular-sieve towers and one of the gas pressure sensors, and the air line is provided with the other of the gas pressure sensors; the oxygen generation line and the air line are connected in parallel to each other and then connected to the vent line, and the vent line is provided with the gas flow regulation valve, the gas flow sensor and the oxygen concentration sensor; and the multi-way valve controls air compressed by the compressor to enter at least one of the oxygen generation line and the air line.

15. The liver hypothermic oxygenated perfusion preservation device according to claim 14, **characterised in that** the oxygen generation line and the air line are each provided with a gas buffer chamber.

16. The liver hypothermic oxygenated perfusion preservation device according to claim 14, **characterised in that** the portal vein perfusion line is provided with a liquid analysis unit, the liquid analysis unit determining at least a partial pressure of oxygen of the perfusate.

17. A hypothermic perfusion method for a liver hypothermic perfusion preservation device according to any one of claims 1-13, the method comprising:
supplying, by a main pump, a perfusate from a main reservoir to a portal vein and a hepatic artery of an isolated liver respectively by means of a portal vein perfusion line and a hepatic artery perfusion line at a flow rate; and
measuring a pressure of the hepatic artery perfusion line, and when the pressure exceeds a set value, and controlling, by a controller, a valve on the hepatic artery perfusion line to be closed to stop the main reservoir from supplying the perfusate to the hepatic artery, while controlling an auxiliary pump to independently supply the perfusate from an auxiliary reservoir to the hepatic artery by means of a hepatic artery perfusion branch.

18. The hypothermic perfusion method according to claim 17, **characterised in that** the perfusate in the auxiliary reservoir is supplied from the main reservoir.

19. The hypothermic perfusion method according to claim 18, **characterised in that** when a liquid level of the perfusate in the auxiliary reservoir is at a low level, the controller controls all valves on the hepatic artery perfusion line, the hepatic artery perfusion branch and the portal vein perfusion line to be closed, and when the liquid level of the perfusate in the auxiliary reservoir rises to a high level, the controller controls the valves on the hepatic artery perfusion branch and the portal vein perfusion line to be opened to restart perfusion of the hepatic artery and the portal vein.

20. The hypothermic perfusion method according to claim 17, **characterised in that** when a liquid level of the perfusate in the main reservoir is at a medium level, the controller controls both valves on the hepatic artery perfusion line and the portal vein perfusion line to be closed, and when the liquid level of the perfusate in the main reservoir rises to a high level, the controller controls the valves on the hepatic artery perfusion line and the portal vein perfusion line to be opened to restart perfusion of the hepatic artery and the portal vein.

21. The hypothermic perfusion method according to claim 17, **characterised in that** the auxiliary pump generates a pulsating flow.

22. The hypothermic perfusion method according to claim 17, **characterised in that** the auxiliary pump generates a continuous flow.

23. The hypothermic perfusion method according to claim 17, **characterised in that** when a bubble sensor on the hepatic artery perfusion line or on the portal vein perfusion line senses a bubble, valves on the hepatic artery perfusion line and the portal vein perfusion line are controlled to be closed to stop perfusing the hepatic artery and the portal vein, and the perfusate is discharged from an irrigation line in communication with the hepatic artery perfusion line and the portal vein perfusion line.

24. The hypothermic perfusion method according to claim 17, **characterised by** further comprising an irrigation mode in which the controller controls valves on the hepatic artery perfusion line, the hepatic artery perfusion branch and the portal vein perfusion line to be closed, the perfusate flows into the main reservoir, and when a liquid level of the perfusate in the main reservoir reaches a high level, the irrigation line is opened, and the perfusate is discharged from the irrigation line; and
the controller controls the perfusate to flow from the main reservoir into the auxiliary reservoir after the liquid level of the perfusate in the main reservoir is kept at the high level for a period of time, and when a liquid level of the perfusate in the auxiliary reservoir reaches a high level, the irrigation line is opened, and the perfusate is discharged from the irrigation line.

25. The hypothermic perfusion method according to claim 17, **characterised by** further comprising an exhaust mode in which
the hepatic artery of the isolated liver and the hepatic artery perfusion line are connected to two ends of a three-way cannula respectively while the remaining tail end is kept in an open state, and the portal vein of the isolated liver and the portal vein perfusion line are connected to two ends of a further three-way cannula respectively while the remaining tail end is kept in an open state; and
the main pump is controlled to run, the perfusate flows through the hepatic artery perfusion line and the portal vein perfusion line, and flows out from the tail ends of the three-way cannulas on the respective lines, to discharge a gas from the lines, and the tail ends of the three-way cannulas are sealed with covers as the perfusate flows out, so as to end the exhaust mode.

26. The hypothermic perfusion method according to claim 17, **characterised by** further comprising a step of determining a flow rate of the main pump, wherein the controller monitors the time taken by the main reservoir from a low liquid level to a high liquid level as P, a volume of the main reservoir from the low liquid level to the high liquid level is Q, an actual output flow rate of the main pump is calculated as Q/P, and Q/P is compared with a theoretical flow rate value of the main pump to determine whether the flow rate of the main pump is accurate.

27. The hypothermic perfusion method according to claim 17, **characterised by** further comprising a step of determining a flow rate of the auxiliary pump, wherein the controller monitors the time taken by the auxiliary reservoir from a high liquid level to a low liquid level as P, a volume of the auxiliary reservoir from the low liquid level to the high liquid level is Q, an actual output flow rate of the auxiliary pump is calculated as Q/P, and Q/P is compared with a theoretical flow rate value of the auxiliary pump to determine whether the flow rate of the auxiliary pump is accurate.

28. A method for operating a molecular-sieve oxygen generation unit of a liver hypothermic perfusion preservation device according to any one of claims 14 to 16, the method comprising the following steps:
step I of controlling a compressor to run by a controller, wherein air is compressed by the compressor to become compressed air;
step II of controlling a multi-way valve to work by the controller, wherein the compressed air enters a first molecular-sieve tower for nitrogen adsorption, and oxygen flowing out from the first molecular-sieve tower enters an oxygenator through an oxygen generation line and a vent line; and
step III of controlling by the controller, after the first molecular-sieve tower works for a period of time, the multi-way valve to cause the compressed air to enter a second molecular-sieve tower, wherein oxygen flowing out from the molecular-sieve tower is partially supplied to the oxygenator and partially blown back to the first molecular-sieve tower such that nitrogen in the first molecular-sieve tower is discharged from the molecular-sieve oxygen generation unit, and step II is restarted.

29. The method for operating a molecular-sieve oxygen generation unit according to claim 28, **characterised in that** a gas flow regulation valve on the vent line automatically regulates an actual flow rate to a target flow rate according to a pressure measured by a gas pressure sensor on the oxygen generation line.

30. The method for operating a molecular-sieve oxygen generation unit according to claim 28, **characterised in that** when it is necessary to rapidly reduce a partial pressure of oxygen, the controller controls the multi-way valve to work, and the compressed air enters the oxygenator through an air line and the vent line.
